# EUROPEAN PATENT SPECIFICATION

(11) **EP 2 926 851 B1**
(45) Date of publication and mention of the grant of the patent: **10.07.2019**
(21) Application number: 15154433.5
(22) Date of filing: 10.02.2015
(51) Int. Cl.: A61M 5/315, A61L 31/04, B29D 99/00, B29K 105/00

(54) **Gasket for prefilled syringe, and production method therefor**
Dichtung für eine vorgefüllte Spritze und Herstellungsverfahren dafür
Joint d'étanchéité pour seringue pré-remplie et son procédé de production

(30) Priority: 31.03.2014 JP 2014073761
(43) Date of publication of application: 07.10.2015
(73) Proprietor: Sumitomo Rubber Industries, Ltd., Kobe-shi, Hyogo-ken 651-0072 (JP)
(72) Inventor: Kaneko, Hiroyuki, Kobe-shi, Hyogo 651-0072 (JP); Nakano, Hiroaki, Kobe-shi, Hyogo 651-0072 (JP); Hara, Seiji, Kobe-shi, Hyogo 651-0072 (JP)
(74) Representative: Manitz Finsterwald Patent- und Rechtsanwaltspartnerschaft mbB

(56) References cited:
- EP-A1- 2 226 088
- EP-A2- 1 870 117
- JP-A- 2003 245 350
- JP-A- 2004 008 509
- JP-B2- 4 708 013
- US-A1- 2005 218 548
- US-A1- 2013 053 786

## Description

### TECHNICAL FIELD

The present invention relates to a gasket for a prefilled syringe, and a production method therefor.

### BACKGROUND ART

In recent years, syringes prefilled with a liquid drug have been increasingly used because of their handling ease and their capability of preventing medical accidents such as administration of a wrong drug (see Patent Document 1). A distal end of such a prefilled syringe to which an injection needle is attached is sealed with a cap. For administration of the liquid drug, the injection needle is attached to the distal end, and a plunger rod is pushed toward the distal end to slide a gasket of the prefilled syringe. Thus, the liquid drug is administered through the injection needle.

Since rubber members such as the gasket and the cap used in the prefilled syringe are kept in direct contact with the liquid drug for a long period of time, butyl rubber materials excellent in chemical resistance, gas permeation resistance, water vapor permeation resistance and anti-aging property are often used for the rubber members.

However, bioengineered drugs and the like are liable to be adversely affected by the gasket and the nozzle cap through interactions between the drug and a raw rubber and other ingredients of a rubber material leached from the gasket and the cap and a coating separated from the gasket and the cap.

Particularly, a distal end face of the gasket directly contacts the liquid drug contained in a barrel of the syringe, so that the raw rubber and other ingredients of the rubber material are liable to be leached into the liquid drug from a body of the gasket.

A sliding portion of the gasket kept in contact with an inner peripheral surface of the syringe barrel is less liable to be brought into direct contact with the liquid drug, but is coated with an oil type silicone lubricant or a cured type silicone lubricant in order to improve the slidability of the gasket with respect to the inner peripheral surface of the barrel when the gasket is fitted in the barrel or when the plunger is pushed for injection of the liquid drug. Therefore, the liquid drug is liable to be contaminated with the silicone as foreign matter, so that the quality of the liquid drug is adversely affected by the silicone depending on the type of the liquid drug. Particularly, the inner peripheral surface of the syringe barrel is coated with a greater amount of the silicone coating agent than the gasket, and has a greater area of contact with the liquid drug contained in the syringe barrel. This significantly influences the liquid drug.

In order to ensure the stability of the drug, a laminated gasket product has recently been developed, which includes a gasket body of an elastic material such as a butyl rubber laminated with a fluororesin film having an excellent chemical resistance. It has been proposed to use the laminated gasket for glass syringes and prefilled resin syringes.

According to the proposal, the rubber component is prevented from being leached into the liquid drug from the distal end face of the gasket kept in direct contact with the liquid drug, and the sliding resistance of the side surface (circumferential surface) of the gasket kept in contact with the inner surface of the syringe barrel is reduced. This obviates the need for coating the inner surface of the syringe barrel with a great amount of the silicone coating agent, thereby preventing the contamination of the liquid drug due to separation of the silicone coating from the inner surface of the barrel. That is, the safety of the liquid drug contained in the syringe is enhanced by laminating at least the outer surface of the gasket body with the fluororesin film.

Further, it is possible to obviate the step of coating the inner surface of the syringe barrel with a great amount of silicone, thereby advantageously reducing the costs for the assembling of the syringe.

JP 2003-245,350 A discloses a gasket for a syringe having a liquid contacting portion and a sliding portion, which keeps contact with the inner surface of a syringe barrel. Moreover, after the gasket is placed in the syringe barrel, the gasket is rotated and thereby the surface of the sliding portion of the formed gasket is rubbed against the surface of the syringe barrel. Furthermore, by rotating the gasket, the rubbing direction against which the surface of the sliding portion is rubbed in the rubbing step extends circumferentially to the sliding portion. Further prior art is cited in the following documents US2013/0053786 A1, US 2005/218548 A1, US6, 986, 705, and JP 2012-147859 A, JP 2003 245350 A, JP 4 708013 B2.

### CITATION LIST

### PATENT DOCUMENT

PATENT DOCUMENT 1: JP2005-185747-A

### SUMMARY OF THE INVENTION

### PROBLEM TO BE SOLVED BY THE INVENTION

The chemical resistance can be improved by providing an inert film such as of the fluororesin on the surface of the gasket (by laminating the gasket with the inert film). However, the inert film is liable to be scratched in a film production process or a laminated gasket production process. If the gasket laminated with the scratched film is used, the liquid drug is liable to leak through the scratch. Particularly, where the scratch extends parallel to a gasket sliding direction, the leakage of the liquid drug is more liable to occur.

To solve the problem described above, it is a principal object of the present invention to provide a gasket for a prefilled syringe and a method for producing the gasket, which ensure that a sliding portion of the laminated gasket (a portion of the laminated gasket to be kept in contact with a syringe barrel) is substantially free from a scratch or an existing scratch of the laminated gasket is substantially eliminated.

### SOLUTION TO PROBLEM

According to an invention aspect, there is provided a method for producing a prefilled syringe gasket including a liquid contact portion and a sliding portion to be kept in contact with an inner surface of a syringe barrel, the method including the steps of forming the gasket, and rubbing a surface of the sliding portion of the formed gasket against a predetermined base, wherein a rubbing direction in which the surface of the sliding portion is rubbed in the rubbing step extends circumferentially of the sliding portion, wherein the step of rubbing is mechanically performed at a constant speed for a predetermined period by means of a rotating rotor, and wherein the sliding portion of the gasket has an arithmetic average roughness R_{a//} as measured in a circumferential direction thereof and an arithmetic average roughness R_{a⊥} as measured in a direction perpendicular to the circumferential direction thereof in a sliding direction, and the ratio R_{a⊥}/R_{a//} of the arithmetic average roughness R_{a⊥} to the arithmetic average roughness R_{a//} is not less than 1.00.

According to another inventive aspect, there is provided a prefilled syringe gasket, which includes a liquid contact portion, and a sliding portion to be kept in contact with an inner surface of a syringe barrel, wherein the sliding portion of the gasket has an arithmetic average roughness R_{a//} as measured in a circumferential direction thereof and an arithmetic average roughness R_{a⊥} as measured in a direction perpendicular to the circumferential direction thereof (in a sliding direction), and the ratio (R_{a⊥}/R_{a//}) of the arithmetic average roughness R_{a⊥} to the arithmetic average roughness R_{a//} is not less than 1.00.

According to still another inventive aspect, there is provided a medical syringe, which includes a hollow cylindrical syringe barrel, a plunger combined with the syringe barrel and movable in the syringe barrel, and a prefilled syringe gasket attached to a distal end of the plunger.

### EFFECTS OF THE INVENTION

The present invention provides a laminated gasket which is free from leakage of a liquid drug contained in a prefilled syringe.

### BRIEF DESCRIPTION OF THE DRAWINGS

FIG. 1 is an exploded diagram illustrating a medical syringe according to an embodiment of the present invention.
FIG. 2 is a diagram of a laminated gasket according to the embodiment of the present invention with a half of the gasket illustrated in section.

### EMBODIMENT OF THE INVENTION

With reference to the attached drawings, one embodiment of the present invention will hereinafter be described specifically.

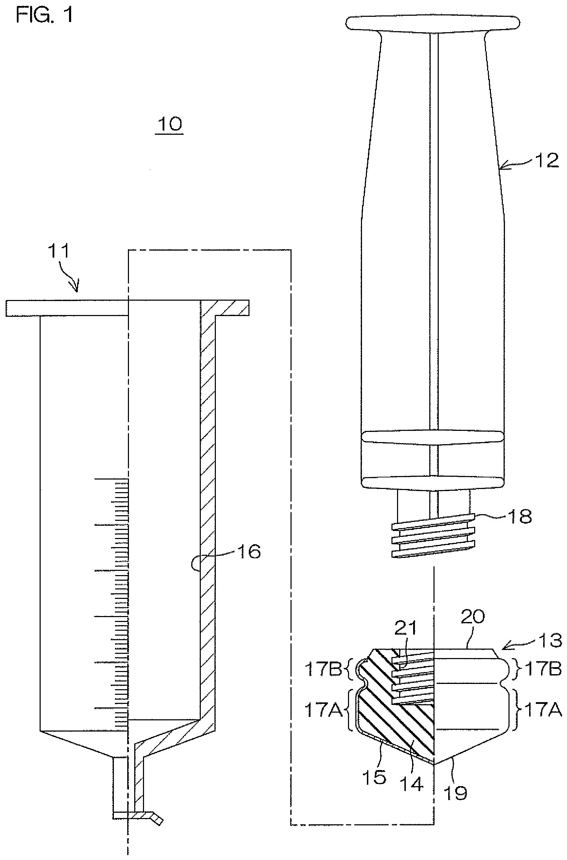

FIG. 1 is an exploded diagram illustrating a medical syringe, i.e., a so-called prefilled syringe, according to the embodiment of the present invention. In FIG. 1, a half of a syringe barrel 11 and a half of a gasket 13 are illustrated in section.

Referring to FIG. 1, the prefilled syringe 10 includes a hollow cylindrical syringe barrel 11, a plunger 12 combined with the syringe barrel 11 and reciprocally movable in the syringe barrel 11, and a gasket 13 attached to a distal end of the plunger 12. The gasket 13 is a so-called laminated gasket, which includes a main body 14 made of an elastic material (a rubber or an elastomer) and a lamination film 15 provided on a surface of the main body 14. The gasket 13 includes two circumferential portions 17A, 17B serving as a sliding portion to be kept in air-tight and liquid-tight contact with an inner peripheral surface 16 of the syringe barrel 11.

The plunger 12 is a resin plate piece, for example, having a cross shape as seen in section, and includes a head 18 provided at its distal end to which the gasket 13 is attached. The head 18 is an integral part of the plunger 12 made of a resin and shaped in a male screw.

The gasket 13 has a generally cylindrical shape having a short axis. The gasket 13 has a distal end face, for example, having a conical center portion projecting at an obtuse angle, and a rear end face axially recessed into an engagement recess 21 shaped in a female screw. The head 18 of the plunger 12 is screwed into the engagement recess 21 of the gasket 13, whereby the gasket 13 is attached to the distal end of the plunger 12.

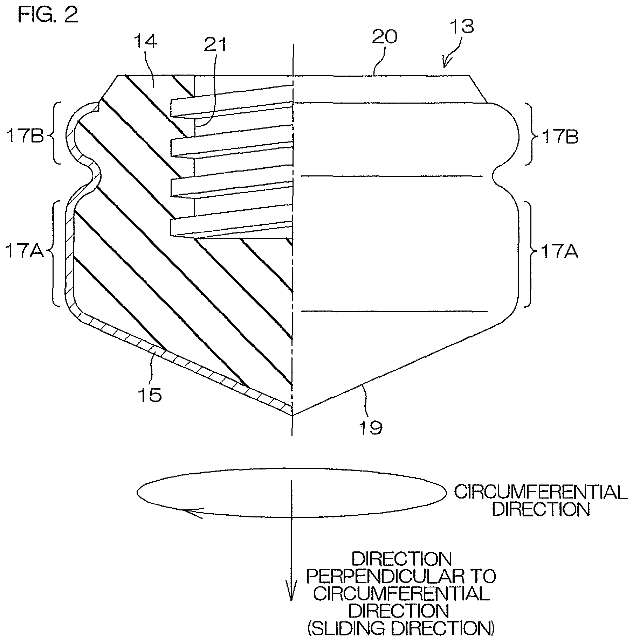

FIG. 2 is a diagram showing only the gasket 13 of FIG. 1 on an enlarged scale. In FIG. 2, a half of the gasket 13 is illustrated in section.

Referring to FIG. 2, the structure of the gasket 13 according to this embodiment will be described in detail.

The gasket 13 includes the main body 14, and the lamination film 15 provided on the surface of the main body 14. The main body 14 is formed from a rubber (elastic material).

The type of the lamination film 15 provided on the surface of the main body 14 is not particularly limited, as long as the lamination film 15 can prevent migration of components from a crosslinked rubber (main body 14) and is more slidable than the rubber, i.e., has a smaller friction coefficient than the rubber.

Exemplary materials for the main body 14 and the lamination film 15 are shown below.

### <Rubber>

Examples of the rubber as the material for the main body 14 include butyl rubbers, isoprene rubbers, butadiene rubbers, styrene-butadiene rubbers, natural rubbers, chloroprene rubbers, nitrile rubbers such as acrylonitrile-butadiene rubbers, hydrogenated nitrile rubbers, norbornene rubbers, ethylene-propylene rubbers, ethylene-propylene-diene rubbers, acryl rubbers, ethylene-acrylate rubbers, fluororubbers, chlorosulfonated polyethylene rubbers, epichlorohydrin rubbers, silicone rubbers, urethane rubbers, polysulfide rubbers, phosphazene rubbers and 1,2-polybutadiene rubbers.

These rubbers may be used either alone or in combination. The rubber to be used for the main body 14 is not limited to the aforementioned rubbers, but is preferably a butyl rubber and/or an ethylene-propylene-diene rubber (hereinafter referred to as EPDM rubber).

The butyl rubber is preferred because of its excellent gas permeation resistance and water vapor permeation resistance.

A known butyl rubber compound may be used as the butyl rubber, but other examples of the butyl rubber include an isobutylene-isoprene copolymer rubber, a halogenated isobutylene-isoprene copolymer rubber (hereinafter referred to as halogenated butyl rubber), and modification products of any of these rubbers. Examples of the modification products include bromination products of copolymers of isobutylene and p-methylstyrene. Particularly, the halogenated butyl rubber is more preferable, and a chlorinated butyl rubber and a brominated butyl rubber are further more preferable for easy crosslinking.

The EPDM rubber is preferred because of its excellent processability. The EPDM rubber includes a non-oil extension type EPDM rubber containing only a rubber component and an oil extension type EPDM rubber containing a rubber component and an extension oil. In the present invention, either type of the EPDM rubbers may be used. Examples of a diene monomer for the EPDM rubber include dicyclopentadiene, methylene norbornene, ethylidene norbornene, 1,4-hexadiene and cyclooctadiene.

The halogenated butyl rubber and the EPDM rubber are advantageously used in combination because the resulting rubber is excellent in gas permeation resistance, water vapor permeation resistance and processability.

In this embodiment, the main body 14 is of the syringe gasket as a medical rubber product and, therefore, a butyl rubber having a lower gas permeability is preferably used as a principal rubber component. A triazine derivative crosslinking agent is preferably used as a crosslinking agent for cleanliness.

### <Lamination film 15>

The lamination film 15 is not particularly limited, but may be an inert film. At least one fluororesin selected from the group consisting of a polytetrafluoroethylene (PTFE), a modified polytetrafluoroethylene (modified PTFE, which is a copolymer of a 4F-monomer and a very small amount of a perfluoroalkoxide), a tetrafluoroethylene ethylene copolymer (ETFE), a tetrafluoroethylene perfluoroalkyl vinyl ether copolymer (PFA) and a polychlorotetrafluoroethylene (PCTFE), and/or an olefin resin is preferred for the lamination film 15 to provide excellent chemical resistance.

### <Step of Forming Laminated Gasket 13>

The laminated gasket 13 according to this embodiment is produced by kneading ingredients blended in a predetermined blend radio by means of a sealed kneader, an open roll kneader or the like, forming the resulting kneaded product into an unvulcanized rubber sheet by means of a calender or a sheet forming machine, placing the unvulcanized rubber sheet and the inert film each having a predetermined weight and size in superposition in a die, and press-forming the unvulcanized rubber sheet and the inert film into a laminated gasket sheet by means of a vacuum press.

Conditions for the forming are not particularly limited, but may be properly determined. The forming temperature is preferably 155°C to 200°C, more preferably 165°C to 180°C. The forming period is preferably 1 to 20 minutes, more preferably 3 to 15 minutes, further more preferably 5 to 10 minutes. The die to be used for the forming preferably has a sliding surface forming portion having a smooth die surface mirror-finished so as to have an arithmetic average roughness Rₐ of not greater than 0.03 µm as measured with a cutoff value of 0.08 mm. With the use of this die, the laminated rubber member thus formed can have a smaller surface roughness than the original surface roughness of the inert film. The arithmetic average roughness Rₐ is preferably not greater than 0.02 µm, more preferably not greater than 0.015 µm.

Thereafter, an unnecessary portion is cut away and removed from the formed gasket sheet. Then, the resulting product is cleaned, sterilized and dried, and then visually inspected. Thus, a primary gasket product is produced.

A feature of the laminated gasket 13 according to this embodiment is that the primary gasket product includes two circumferential portions 17A, 17B as the sliding portion to be kept in gas-tight and liquid-tight contact with the inner peripheral surface 16 of the syringe barrel 11 and a surface of a forward one 17A of the circumferential portions 17A, 17B of the primary gasket product is rubbed against (polished with) a predetermined base in a circumferential direction.

In this embodiment, the two circumferential portions 17A, 17B, i.e., the forward circumferential portion 17A and the rearward circumferential portion 17B, are provided as the sliding portion, but only one circumferential portion 17A may be provided or three or more sliding portions may be provided. The shape of the gasket is not limited to the aforementioned shape of this embodiment.

Next, the step of rubbing the primary gasket product will be described.

### <Rubbing Step and Base for Use in Rubbing Step>

The base is not particularly limited, but may be an organic base such as of a natural polymer or a synthetic polymer, an inorganic base such as of a metal or a ceramic material, or an inorganic-organic composite base. More preferably, a fabric formed from a natural polymer or a synthetic polymer or sand paper is preferably used as the base.

The rubbing step is mechanically performed at a constant speed for a predetermined period by means of a rotating rotor.

### <Gasket>

An exemplary method for judging if the number of scratches present in the sliding portion and extending in the sliding direction is reduced in the present invention is such that an arithmetic average roughness R_{a//} as measured in the circumferential direction of the gasket sliding portion and an arithmetic average roughness R_{a⊥} as measured in a direction (= the sliding direction) perpendicular to the circumferential direction are determined after the step of rubbing the gasket sliding portion against the base. If the scratches extending in the sliding direction are present, the arithmetic average roughness measured in the circumferential direction is greater than the arithmetic average roughness measured in the sliding direction and, therefore, the ratio (R_{a⊥}R_{a//}) of the arithmetic average roughness measured in the sliding direction to the arithmetic average roughness measured in the circumferential direction is smaller than 1.00. If the number of the scratches extending in the sliding direction is reduced by the present invention, the sliding portion has a smooth surface as observed in the circumferential direction, and has a smaller arithmetic average roughness as measured in the circumferential direction. Therefore, the ratio (R_{a⊥/}R_{a//}) of the arithmetic average roughness measured in the sliding direction to the arithmetic average roughness measured in the circumferential direction is not smaller than 1.00.

In the present invention, a difference between a maximum height R_{y//} as measured in the circumferential direction and a maximum height R_{y⊥} as measured in the direction (sliding direction) perpendicular to the circumferential direction (a difference ΔR_{y} = | (R_{y//} - R_{y⊥}) | (wherein | | indicates an absolute value and, therefore, ΔR_{y} ≥ 0)) is not greater than 0.100 µm, preferably 0.080 µm, more preferably 0.06 µm.

If ΔR_{y} is greater than 0.100 µm, the gasket does not uniformly contact the barrel and, therefore, leakage of the liquid drug is more liable to occur.

The present invention will hereinafter be described in greater detail by way of examples thereof. It should be understood that the present invention be not limited to the following examples.

### EXAMPLES

### <Production Method 1>

An unvulcanized rubber sheet containing a chlorinated butyl rubber was combined with a 100-µm thick inert film, and vulcanized at 175°C for 10 minutes in a vacuum press to be thereby bonded to the inert film. Thus, the rubber sheet and the inert film were press-formed into a sheet having a plurality of gasket-shaped pieces. The sheet thus formed was stamped, whereby gaskets each having a shape as shown in FIG. 2 were produced. The resulting gaskets were cleaned, sterilized and dried. Thus, gaskets A were produced.

### <Production Method 2>

The gaskets produced in the production method 1 were each fixed to a distal end of an electric router (mini router available from Proxxon GmbH), and pressed against a cotton fabric (MK0012 available from Taenaka Pile Fabrics Co., Ltd.), whereby the sliding portion of each of the gaskets was rubbed against the cotton fabric in a circumferential direction at a constant speed (8000 rpm) for a predetermined period (5 seconds) . The resulting gaskets were immersed in pure water, and cleaned by means of an ultrasonic cleaning machine. Thereafter, the gaskets were sterilized and dried. Thus, gaskets B were produced.

### <Production Method 3>

Gaskets C were produced by processing the gaskets in substantially the same manner as in Production Method 2, except that the gaskets were each pressed against the cotton fabric for 30 seconds in Production Method 2.

### <Production Method 4>

Gaskets D were produced by processing the gaskets in substantially the same manner as in Production Method 2, except that the gaskets were each pressed against the cotton fabric for 60 seconds in Production Method 2.

### <Production Method 5>

Gaskets E were produced by processing the gaskets in substantially the same manner as in Production Method 2, except that a rayon fabric (YA-18-R available from Yoshikawa Chemical Co., Ltd. and having a filament diameter of 2.5 D) was used as the base and the gaskets were each pressed against the rayon fabric for 5 seconds in Production Method 2.

### <Production Method 6>

Gaskets F were produced by processing the gaskets in substantially the same manner as in Production Method 2, except that the gaskets were each pressed against the rayon fabric for 30 seconds in Production Method 5.

### <Production Method 7>

Gaskets G were produced by processing the gaskets in substantially the same manner as in Production Method 2, except that the gaskets were each pressed against the rayon fabric for 60 seconds in Production Method 5.

The following tests were performed on the gaskets produced by the aforementioned production methods.

### <Liquid Drug Sealability Test>

An aqueous solution containing 0.1 wt% of a nonionic surfactant (POLYSOLVATE 80) was prepared as a liquid to be sealed in the prefilled syringe. The gasket was attached to a plastic syringe (of a cyclic olefin polymer) having a volume of 1 mL and an inner diameter of 6.35 mm, and the aqueous solution was injected into the syringe. Then, a distal end of the syringe was capped with a rubber cap, and the resulting syringe sample was allowed to stand in an oven at 40°C for one week. Thereafter, the sample was taken out of the oven, and a portion of the gasket present between the two sliding portions (hereinafter referred to as "trough" between the circumferential portions 17A and 17B) was visually inspected at a magnification of 50X by means of a video microscope (DVM5000 available from Leica Microsystems GmbH) to see whether the solution reached the trough. Where the solution reached the trough, it was determined that liquid leakage occurred.

### <Measurement of Surface Roughnesses of Sliding Portion>

The surface roughnesses of the circumferential portion 17A serving as the gasket sliding portion were measured by means of a laser microscope (VK-X100 available from Keyence Corporation). The measurement was performed at a comprehensive magnification of 1000X, and arithmetic average roughnesses Rₐ and maximum heights R_{y} of the sliding portion 17A were measured in a direction perpendicular to a circumferential direction of the sliding portion 17A (in a longitudinal direction) and a direction parallel to the circumferential direction of the sliding portion 17A (in a transverse direction) (in conformity with JIS B06014:1994 with a cutoff of 0.25 mm).

### <Example 1>

The surface roughnesses of the sliding portion 17A of the gasket C produced in Production Method 3 were measured. As a result, the gasket C had an arithmetic average roughness of 0.077 µm as measured in the direction perpendicular to the circumferential direction and an arithmetic average roughness of 0.071 µm as measured in the circumferential direction. Further, the gasket C had a maximum height of 0.541 µm as measured in the direction perpendicular to the circumferential direction and a maximum height of 0.532 µm as measured in the circumferential direction. Further, the liquid drug sealability test was performed on 50 such gaskets C. As a result, the liquid leakage was not observed.

### <Example 2>

The surface roughnesses of the sliding portion 17A of the gasket D produced in Production Method 4 were measured. As a result, the gasket D had an arithmetic average roughness of 0.074 µm as measured in the direction perpendicular to the circumferential direction and an arithmetic average roughness of 0.067 µm as measured in the circumferential direction. Further, the gasket D had a maximum height of 0.536 µm as measured in the direction perpendicular to the circumferential direction and a maximum height of 0.503 µm as measured in the circumferential direction. Further, the liquid drug sealability test was performed on 50 such gaskets D. As a result, the liquid leakage was not observed.

### <Example 3>

The surface roughnesses of the sliding portion 17A of the gasket F produced in Production Method 6 were measured. As a result, the gasket F had an arithmetic average roughness of 0.076 µm as measured in the direction perpendicular to the circumferential direction and an arithmetic average roughness of 0.071 µm as measured in the circumferential direction. Further, the gasket F had a maximum height of 0.536 µm as measured in the direction perpendicular to the circumferential direction and a maximum height of 0.561 µm as measured in the circumferential direction. Further, the liquid drug sealability test was performed on 50 such gaskets F. As a result, the liquid leakage was not observed.

### <Example 4>

The surface roughnesses of the sliding portion 17A of the gasket G produced in Production Method 7 were measured. As a result, the gasket G had an arithmetic average roughness of 0.072 µm as measured in the direction perpendicular to the circumferential direction and an arithmetic average roughness of 0.066 µm as measured in the circumferential direction. Further, the gasket G had a maximum height of 0.531 µm as measured in the direction perpendicular to the circumferential direction and a maximum height of 0.558 µm as measured in the circumferential direction. Further, the liquid drug sealability test was performed on 50 such gaskets G. As a result, the liquid leakage was not observed.

### <Comparative Example 1>

The surface roughnesses of the sliding portion 17A of the gasket A produced in Production Method 1 were measured. As a result, the gasket A had an arithmetic average roughness of 0.078 µm as measured in the direction perpendicular to the circumferential direction and an arithmetic average roughness of 0.096 µm as measured in the circumferential direction. Further, the gasket A had a maximum height of 0.537 µm as measured in the direction perpendicular to the circumferential direction and a maximum height of 0.658 µm as measured in the circumferential direction. Further, the liquid drug sealability test was performed on 50 such gaskets A. As a result, six of the gaskets A suffered from the liquid leakage.

### <Comparative Example 2>

The surface roughnesses of the sliding portion 17A of the gasket B produced in Production Method 2 were measured. As a result, the gasket B had an arithmetic average roughness of 0.076 µm as measured in the direction perpendicular to the circumferential direction and an arithmetic average roughness of 0.092 µm as measured in the circumferential direction. Further, the gasket B had a maximum height of 0.536 µm as measured in the direction perpendicular to the circumferential direction and a maximum height of 0.644 µm as measured in the circumferential direction. Further, the liquid drug sealability test was performed on 50 such gaskets B. As a result, five of the gaskets B suffered from the liquid leakage.

### <Comparative Example 3>

The surface roughnesses of the sliding portion 17A of the gasket E produced in Production Method 5 were measured. As a result, the gasket E had an arithmetic average roughness of 0.078 µm as measured in the direction perpendicular to the circumferential direction and an arithmetic average roughness of 0.094 µm as measured in the circumferential direction. Further, the gasket E had a maximum height of 0.532 µm as measured in the direction perpendicular to the circumferential direction and a maximum height of 0.644 µm as measured in the circumferential direction. Further, the liquid drug sealability test was performed on 50 such gaskets E. As a result, five of the gaskets E suffered from the liquid leakage.

The results are collectively shown in Table 1.

**Table 1**

| | Example 1 | Example 2 | Example 3 | Example 4 | Comparative Example 1 | Comparative Example 2 | Comparative Example 3 |
|---|---|---|---|---|---|---|---|
| Gasket used | C | D | F | G | A | B | E |
| Liquid drug sealability test | | | | | | | |
| Leakage | No | No | No | No | Yes | Yes | Yes |
| | 0/50 | 0/50 | 0/50 | 0/50 | 6/50 | 5/50 | 4/50 |

| Arithmetic average roughnesses | | | | | | | |
|---|---|---|---|---|---|---|---|
| In direction perpendicular to circumferential direction (R_{a⊥}) [µm] | 0.077 | 0.074 | 0.076 | 0.072 | 0.078 | 0.076 | 0.078 |
| In circumferential direction (R_{a//}) [µm] | 0.071 | 0.067 | 0.071 | 0.066 | 0.096 | 0.092 | 0.094 |
| Radio (R_{a⊥}/ R_{a//}) | 1.08 | 1.10 | 1.07 | 1.09 | 0.81 | 0.83 | 0.83 |

| Maximum heights | | | | | | | |
|---|---|---|---|---|---|---|---|
| In direction perpendicular to circumferential direction (R_{y⊥}) [µm] | 0.541 | 0.536 | 0.536 | 0.531 | 0.537 | 0.536 | 0.532 |
| In circumferential direction (R_{y//}) [µm] | 0.532 | 0.503 | 0.561 | 0.558 | 0.658 | 0.644 | 0.644 |
| ΔR_{y} = \| R_{y⊥} - R_{y//} \| | 0.009 | 0.033 | 0.025 | 0.027 | 0.121 | 0.108 | 0.112 |

As shown in Table 1, none of the 50 gaskets of each of Examples 1 to 4 subjected to the liquid drug sealability test suffered from the leakage. On the other hand, 6 to 4 of the 50 gaskets of each of Comparative Examples 1 to 3 suffered from the leakage.

### DESCRIPTION OF REFERENCE CHARACTERS

- 10: PREFILLED SYRINGE
- 11: SYRINGE BARREL
- 12: PLUNGER
- 13: GASKET
- 17A, 17B: CIRCUMFERENTIAL PORTIONS (SLIDING PORTION)

## Claims

1. A method for producing a prefilled syringe gasket (13) including a liquid contact portion and a sliding portion to be kept in contact with an inner surface (16) of a syringe barrel (11), the method comprising the steps of:
forming the gasket (13); and
rubbing a surface of the sliding portion of the formed gasket (13) against a predetermined base;
wherein a rubbing direction in which the surface of the sliding portion is rubbed in the rubbing step extends circumferentially of the sliding portion,
wherein the step of rubbing is mechanically performed at a constant speed for a predetermined period by means of a rotating rotor,
wherein the sliding portion of the gasket has an arithmetic average roughness R_{a//} as measured in a circumferential direction thereof and an arithmetic average roughness R_{a⊥} as measured in a direction perpendicular to the circumferential direction thereof in a sliding direction, and the ratio R_{a-⊥}/R_{a//} of the arithmetic average roughness R_{a⊥} to the arithmetic average roughness R_{a//} is not less than 1.00.

2. The prefilled syringe gasket (13) producing method according to claim 1, wherein the base is one of an organic base and an inorganic base in the step of rubbing the surface of the sliding portion of the gasket (13) against the base.

3. The prefilled syringe gasket (13) producing method according to claim 2, wherein the base is formed from one of a natural polymer, a synthetic polymer, a metal and a ceramic material.

4. The prefilled syringe gasket (13) producing method according to claim 1, wherein the base is a fabric formed from one of a natural polymer and a synthetic polymer.

5. The prefilled syringe gasket (13) producing method according to claim 4, wherein the fabric is a fabric formed from at least one of cotton, rayon, a polyester, a polyethylene, a polypropylene and an acrylic polymer.

6. A prefilled syringe gasket (13) comprising:
a liquid contact portion; and
a sliding portion to be kept in contact with an inner surface (16) of a syringe barrel (11);
wherein the sliding portion of the gasket (13) has an arithmetic average roughness R_{a//} as measured in a circumferential direction thereof and an arithmetic average roughness R_{a⊥} as measured in a direction perpendicular to the circumferential direction thereof in a sliding direction, and a ratio R_{a⊥}/R_{a//} of the arithmetic average roughness R_{a⊥} to the arithmetic average roughness R_{a//} is not less than 1.00.

7. The prefilled syringe gasket (13) according to claim 6, wherein the sliding portion of the gasket (13) has a maximum height R_{y//} as measured in the circumferential direction and a maximum height R_{y⊥} as measured in the direction perpendicular to the circumferential direction in the sliding direction in the measurement of the surface roughnesses, and a difference ΔR_{y} between the maximum height R_{y//} and the maximum height R_{y⊥} is not greater than 0.100 µm.

8. The prefilled syringe gasket (13) according to claim 7, wherein the maximum height of the sliding portion of the gasket (13) measured in the circumferential direction in the measurement of the surface roughnesses is less than 0.600 µm.

9. A medical syringe (10) comprising:
a hollow cylindrical syringe barrel (11);
a plunger (12) combined with the syringe barrel (11) and movable in the syringe barrel (11); and
a gasket (13) attached to a distal end of the plunger (12), the gasket (13) being a gasket (13) as recited in claim 6.

## Patentansprüche

1. Verfahren zur Herstellung einer Dichtung (13) für eine vorgefüllte Spritze mit einem Flüssigkeitskontaktabschnitt und einem Gleitabschnitt, der mit einer Innenfläche (16) eines Spritzenzylinders (11) in Kontakt zu halten ist, wobei das Verfahren die Schritte umfasst:
Bilden der Dichtung (13); und
Reiben einer Oberfläche des Gleitabschnitts der gebildeten Dichtung (13) gegen einen vorbestimmten Grundkörper;
wobei sich eine Reibrichtung, in der die Oberfläche des Gleitabschnitts bei dem Reibschritt gerieben wird, in der Umfangsrichtung des Gleitabschnitts erstreckt,
wobei der Schritt des Reibens mechanisch mit einer konstanten Geschwindigkeit für einen vorbestimmten Zeitraum mittels eines rotierenden Rotors durchgeführt wird,
wobei der Gleitabschnitt der Dichtung einen arithmetischen Mittenrauwert R_{a//}, gemessen in ihrer Umfangsrichtung, und einen arithmetischen Mittenrauwert R_{a⊥}, gemessen in einer Richtung senkrecht zu ihrer Umfangsrichtung in einer Gleitrichtung, aufweist, und das Verhältnis R_{a⊥}/R_{a//} des arithmetischen Mittenrauwerts R_{a⊥} zu dem arithmetischen Mittenrauwert R_{a//} nicht kleiner als 1,00 ist.

2. Herstellungsverfahren einer Dichtung (13) für eine vorgefüllte Spritze nach Anspruch 1, wobei der Grundkörper bei dem Schritt des Reibens der Oberfläche des Gleitabschnitts der Dichtung (13) gegen den Grundkörper einer von einem organischen Grundkörper und einem anorganischen Grundkörper ist.

3. Herstellungsverfahren einer Dichtung (13) für eine vorgefüllte Spritze nach Anspruch 2, wobei der Grundkörper aus einem natürlichen Polymer, einem synthetischen Polymer, einem Metall und einem keramischen Material gebildet ist.

4. Herstellungsverfahren einer Dichtung (13) für eine vorgefüllte Spritze nach Anspruch 1, wobei der Grundkörper ein Gewebe ist, das aus einem natürlichen Polymer und einem synthetischen Polymer gebildet ist.

5. Herstellungsverfahren einer Dichtung (13) für eine vorgefüllte Spritze nach Anspruch 4, wobei das Gewebe ein Gewebe ist, das aus mindestens einem von Baumwolle, Viskose, einem Polyester, einem Polyethylen, einem Polypropylen und einem Acrylpolymer gebildet ist.

6. Dichtung (13) für eine vorgefüllte Spritze, umfassend:
einen Flüssigkeitskontaktabschnitt; und
einen Gleitabschnitt, der mit einer Innenfläche (16) eines Spritzenzylinders (11) in Kontakt zu halten ist,
wobei der Gleitabschnitt der Dichtung (13) einen arithmetischen Mittenrauwert R_{a//}, gemessen in ihrer Umfangsrichtung, und einen arithmetischen Mittenrauwert R_{a⊥}, gemessen in einer Richtung senkrecht zu ihrer Umfangsrichtung in einer Gleitrichtung, aufweist, und ein Verhältnis R_{a⊥}/R_{a//} des arithmetischen Mittenrauwerts R_{a⊥} zu dem arithmetischen Mittenrauwert R_{a//} nicht kleiner als 1,00 ist.

7. Dichtung (13) für eine vorgefüllte Spritze nach Anspruch 6, wobei der Gleitabschnitt der Dichtung (13) eine maximale Höhe R_{y//}, gemessen in der Umfangsrichtung, und eine maximale Höhe R_{y⊥}, gemessen in der Richtung senkrecht zu der Umfangsrichtung in der Gleitrichtung, bei der Messung der Oberflächenrauwerte aufweist, und eine Differenz ΔR_{y} zwischen der maximalen Höhe R_{y//} und der maximalen Höhe R_{y⊥} nicht größer als 0,100 µm ist.

8. Dichtung (13) für eine vorgefüllte Spritze nach Anspruch 7, wobei die maximale Höhe des Gleitabschnitts der Dichtung (13), gemessen in der Umfangsrichtung bei der Messung der Oberflächenrauwerte, kleiner als 0,600 µm ist.

9. Medizinische Spritze (10), umfassend:
einen hohlen zylindrischen Spritzenzylinder (11);
einen Kolben (12), der mit dem Spritzenzylinder (11) kombiniert und in dem Spritzenzylinder (11) beweglich ist; und
eine Dichtung (13), die an einem distalen Ende des Kolbens (12) befestigt ist, wobei die Dichtung (13) eine Dichtung (13) nach Anspruch 6 ist.

## Revendications

1. Procédé de production d'un joint pour seringue préremplie (13) incluant une portion de contact de liquide et une portion de coulissement devant être maintenue en contact avec une surface intérieure (16) d'un cylindre de seringue (11), le procédé comprenant les étapes consistant à :
former le joint (13) ; et
frotter une surface de la portion de coulissement du joint formé (13) contre une base prédéterminée ;
dans lequel une direction de frottement dans laquelle la surface de la portion de coulissement est frottée dans l'étape de frottement s'étend circonférentiellement par rapport à la portion de coulissement,
dans lequel l'étape de frottement est mécaniquement exécutée à une vitesse constante pour une période prédéterminée au moyen d'un rotor en rotation,
dans lequel la portion de coulissement du joint a une rugosité moyenne arithmétique R_{a//} telle que mesurée dans une direction circonférentielle de celle-ci et une rugosité moyenne arithmétique R_{a⊥} telle que mesurée dans une direction perpendiculaire à la direction circonférentielle de celle-ci dans une direction de coulissement, et le rapport R_{a⊥/}R_{a//} de la rugosité moyenne arithmétique R_{a⊥} sur la rugosité moyenne arithmétique R_{a//} n'est pas inférieur à 1,00.

2. Procédé de production d'un joint pour seringue préremplie (13) selon la revendication 1, dans lequel la base est soit une base organique soit une base non organique dans l'étape consistant à frotter la surface de la portion de coulissement du joint (13) contre la base.

3. Procédé de production d'un joint pour seringue préremplie (13) selon la revendication 2, dans lequel la base est formée à partir d'un matériau parmi un polymère naturel, un polymère synthétique, un métal et un matériau céramique.

4. Procédé de production d'un joint pour seringue préremplie (13) selon la revendication 1, dans lequel la base est un tissu formé soit à partir d'un polymère naturel soit à partir d'un polymère synthétique.

5. Procédé de production d'un joint pour seringue préremplie (13) selon la revendication 4, dans lequel le tissu est un tissu formé à partir d'au moins un matériau suivant : coton, rayonne, polyester, polyéthylène, polypropylène et polymère acrylique.

6. Joint pour seringue préremplie (13) comprenant :
une portion de contact de liquide ; et
une portion de coulissement devant être maintenue en contact avec une surface intérieure (16) d'un cylindre de seringue (11) ;
dans lequel la portion de coulissement du joint (13) a une rugosité moyenne arithmétique R_{a//} telle que mesurée dans une direction circonférentielle de celle-ci et une rugosité moyenne arithmétique R_{a⊥} telle que mesurée dans une direction perpendiculaire à la direction circonférentielle de celle-ci dans une direction de coulissement, et un rapport R_{a⊥/}R_{a//} de la rugosité moyenne arithmétique R_{a⊥} sur la rugosité moyenne R_{a//} n'est pas inférieur à 1,00.

7. Joint pour seringue préremplie (13) selon la revendication 6, dans lequel la portion de coulissement du joint (13) a une hauteur maximum R_{y//} telle que mesurée dans la direction circonférentielle et une hauteur maximum R_{y⊥} telle que mesurée dans la direction perpendiculaire à la direction circonférentielle dans la direction de coulissement lors de la mesure des rugosités de surface, et une différence ΔR_{y} entre la hauteur maximum R_{y//} et la hauteur maximum R_{y⊥} n'est pas supérieure à 0,100 µm.

8. Joint pour seringue préremplie (13) selon la revendication 7, dans lequel la hauteur maximum de la portion de coulissement du joint (13) mesurée dans la direction circonférentielle lors de la mesure des rugosités de surface est inférieure à 0,600 µm.

9. Seringue médicale (10) comprenant :
un cylindre de seringue cylindrique creux (11) ;
un piston (12) combiné au cylindre de seringue (11) et déplaçable dans le cylindre de seringue (11) ; et
un joint (13) attaché à une extrémité distale du piston (12), le joint (13) étant un joint (13) tel que mentionné dans la revendication 6.
